# EUROPÄISCHE PATENTSCHRIFT

(11) **EP 2 410 979 B1**
(45) Veröffentlichungstag und Bekanntmachung des Hinweises auf die Patenterteilung: **21.12.2016**
(21) Anmeldenummer: 10711362.3
(22) Anmeldetag: 23.02.2010
(51) Int. Cl.: A61K 8/49, A61Q 5/02, A61Q 5/12, A61Q 19/10

(54) **FORMULIERUNGEN ENTHALTEND SORBITANCARBONSÄUREESTER**
FORMULATIONS CONTAINING SORBITAN CARBOXYLIC ACID ESTER
COMPOSITIONS CONTENANT DES ESTERS D'ACIDE CARBOXYLIQUE DE SORBITANE

(30) Priorität: 23.03.2009 DE 102009001748
(43) Veröffentlichungstag der Anmeldung: 01.02.2012
(73) Patentinhaber: Evonik Degussa GmbH, 45128 Essen (DE)
(72) Erfinder: HERRWERTH, Sascha, 45134 Essen (DE); PEGGAU, Jörg, 45357 Essen (DE); GRÜNING, Burghard, 45134 Essen (DE); KORTEMEIER, Uta, 45128 Essen (DE); SPRINGER, Oliver, 46485 Wesel (DE)
(86) Internationale Anmeldenummer: PCT/EP2010/052252
(87) Internationale Veröffentlichungsnummer: WO 2010/108738

(56) Entgegenhaltungen:
- EP-A1- 1 762 216
- EP-A2- 1 813 251
- AU-B2- 654 954
- DE-A1-102004 036 067
- JP-A- 2 067 247
- DATABASE CA, [Online] 29. September 2000 (2000-09-29), FUKUSHIMA NORIKO: "Water-soluble rinses for dishwashers", XP002643077, gefunden im CA Database accession no. 133-209704 -& JP 2000 239697 A (EAGLE STAR KK) 5. September 2000 (2000-09-05)
- DATABASE CA, [Online] 18. Mai 1976 (1976-05-18), MIURA TAKESHI ET AL: "Rinsing assistants", XP002643079, gefunden im CA Database accession no. 85-96134 -& JP 51 056809 A (ASAHI DENKA KOGYO KK) 18. Mai 1976 (1976-05-18)
- DATABASE CA, [Online] 27. August 2003 (2003-08-27), MINEMURA TAKESHI ET AL: "Coenzyme Q10-containing emulsions, and manufacture thereof", XP002643081, gefunden im CA Database accession no. 139_202492 -& JP 2003 238396 A (NISSHIN PHARMA INC; NIKKO CHEMICAL CO LTD) 27. August 2003 (2003-08-27)
- DATABASE CA, [Online] 24. Juli 2008 (2008-07-24), MORI TOSHIKI: "Transparent cleansers comprising nonionic surfactants", XP002643078, gefunden im CA Database accession no. 149-183047 -& JP 2008 169123 A (DAI ICHI KOGYO SEIYAKU CO LTD) 24. Juli 2008 (2008-07-24)

## Beschreibung

### Gebiet der Erfindung

Gegenstand der Erfindung sind polyetherfreie Formulierungen zur Reinigung und Pflege von menschlichen oder tierischen Körperteilen enthaltend Sorbitancarbonsäureester und ein weiteres Tensid, dadurch gekennzeichnet, dass der Carbonsäureanteil des Sorbitancarbonsäureesters sich ableitet von einer Carbonsäure enthaltend 6 bis 10 Kohlenstoff-Atome die Sorbitancarbonsäureester eine Hydroxylzahl (OH-Zahl) von größer 350 aufweisen und dadurch dass 1,2 bis 2 Mol Carbonsäure pro Mol Sorbitol, von dem sich der Sorbitancarbonsäureester ableitet, eingeestert sind. Gegestand der Erfindung ist auch die Verwendung dieser Sorbitancarbonsäureester polyetherfreien tensidischen Formulierungen.

### Stand der Technik

Moderne kosmetische Reinigungsprodukte für Haut und Haar, wie beispielsweise Duschbäder und Haarshampoos, bestehen im Wesentlichen aus
- Wasser als wichtigstem Lösemittel,
- Tensiden,
- Viskositätsregulatoren zum Verdicken der Formulierung,
- Lösungsvermittlern (Solubilisatoren) für wasserunlösliche Substanzen,
- Parfümölen,
- Konservierungsstoffen sowie
- Wirkstoffen zur Pflege von Haut und Haaren, wie z.B. Rückfettern.

Typische Tenside in Körperreinigungsmitteln sind anionischer, amphoterer und zwitterionischer Struktur. Zu den anionischen Tensiden zählen insbesondere die Salze verschiedener Kationen (Natrium, Ammonium oder andere) von Laurylsulfat, Laurylethersulfat, Myristylethersulfat, Cocoylglutamate, Lauryl Glucose Carboxylate etc. Als zwitterionische Tenside werden u.a. Cocamidopropylbetain oder Cocoamidopropylsultain eingesetzt. Amphotere Tenside sind insbesondere Amphoacetate wie Natriumcocoamphoacetat oder Dinatriumcocoamphodiacetat.

Typische, nach dem bisherigen Stand der Technik eingesetzte Verdicker sind NaCl, niedermolekulare nichtionische Tenside, wie Cocosnussfettsäuremonoethanolamid/ -diethanolamid und Laureth-3, oder Polymere, hochmolekulare, assoziative, hochethoxylierte Fettderivate, wie z.B. Polyethylenglykol(9000)-hydriertes Glycerinpalmitat.

Als Solubilisator oder auch Lösungsvermittler wird im Sinne der vorliegenden Erfindung eine Substanz bezeichnet, die in der Lage ist, wasserunlösliche Verbindungen möglichst klar in wässrigen Systemen in Lösung zu bringen. Nach allgemein akzeptierter Vorstellung werden bei diesem Vorgang Aggregate wie Mizellen gebildet, in deren Strukturen die hydrophoben Substanzen integriert sind. Optimal ist die Bildung einer "Mikroemulsion", also einer thermodynamisch stabilen Mischung aus Wasser (wässrige Lösung), einem Öl (nicht mit Wasser mischbare Substanz) und einem Solubilisator bzw. Lösungsvermittler. Typische Solubilisatoren sind ethoxylierte Fettderivate.

Parfümöle werden den Formulierungen allgemein zur Verbesserung der olfaktorischen Eigenschaften zugesetzt. Die Akzeptanz durch den Verbraucher spielt hier die wichtigste Rolle. Daneben ist es möglicherweise vorteilhaft, die Eigengerüche verwendeter Rohstoffe mit Parfümölen zu überdecken.

Konservierungsstoffe werden zur mikrobiologischen Stabilisierung eingesetzt. Im Falle einer Kontamination sollen diese Inhaltstoffe das Keimwachstum verhindern und gegebenenfalls auch Keime abtöten. Konservierungsmittel sind in behördlichen Regularien (z.B. EU-Kosmetikverordnung) ausführlich beschrieben und reguliert.

Typische Pflegeadditive sind ethoxylierte Glycerin-Fettsäureester, wie beispielweise PEG-7 Glyceryl Cocoate, oder kationische Polymere, wie beispielsweise Polyquaternium-7. Diese werden auch als Rückfetter bezeichnet. Bei der Hautreinigung werden neben dem lipophilen Schmutz auch hauteigene Lipide durch die verwendeten Tenside abgewaschen. Dieser Effekt wird oft als unangenehm empfunden, die Haut fühlt sich rau und spröde an. Die Haut wird auch als "trocken" bezeichnet, wobei hier jedoch die Abwesenheit von Lipiden gemeint ist.

Sogenannte Rückfettungsmittel werden Körperreinigungsmitteln zugesetzt, damit der beschriebene Entfettungsvorgang vermindert wird. Im Ergebnis kann einerseits der durch das abgewaschene Lipid hervorgerufene Effekt durch das Rückfettungsmittel kompensiert werden, andererseits aber auch die entfettende Wirkung der Formulierung an sich durch den Einsatz des Rückfetters vermindert werden.

Formulierungstechnisch ist es schwierig, Emollients (kosmetische Öle), wie z.B. Isopropylmyristat, zu diesem Zweck einzusetzen, weil diese Öle aufwendig solubilisiert werden müssen. Gebräuchlichere Rückfetter sind daher hydrophilere Produkte, wie z.B.
Polyethylenglykol(7)glycerylmonococoat (TEGOSOFT GC^{®}), die bereits durch den Überschuss der reinigenden Tenside solubilisiert werden. Die Analyse einer Produktdatenbank, welche weltweite Produktinnovationen in Verbrauchermärkten erfasst ("Global New Products Database": Mintel), ergab, dass 29 % aller Hautreinigungsformulierungen im europäischen Markt im Untersuchungszeitraum (9/05 - 9/06) Polyethylenglykol(7)glycerylmonococoat enthielten.

Es wird angenommen, dass der Rückfettungsvorgang beim Abspülen der Formulierung nach der eigentlichen Wäsche stattfindet. Beim Abwaschvorgang mit Wasser wird die vorhandene Lösung soweit verdünnt, bis die kritische Mizellbildungskonzentration unterschritten ist und die in den Tensidaggregaten solubilisierten lipophilen Komponenten unlöslich werden. Mit der Freisetzung der Mizellkomponenten (die lipophilen Rückfetter, die Tenside und Solubilisatoren) werden die Rückfetter wieder unlöslich. Diese lipophilen Substanzen (sowohl hauteigene Lipide als auch Emollients/kosmetische Öle) fallen aus und ziehen auf die Haut auf.

Zu den Anforderungen an die fertige Formulierung gehören also neben der reinigenden Wirkung, ein cremiger Schaum, ein gutes Anschäumverhalten, ein gutes Schaumvolumen, ein Schutz vor dem Austrocknen der Haut und eine gute Pflegeleistung. Zu den Grundanforderungen an die einzelnen Bestandteile gehören Mildheit, insbesondere eine gute Hautverträglichkeit und Verarbeitbarkeit. Es ist vorteilhaft, wenn möglichst viele der Anforderungen an eine kosmetische Formulierung durch möglichst wenige toxikologisch unbedenkliche und universell einsetzbare Bestandteile erfüllt werden könnten.

Des Weiteren besteht ein steigender Bedarf an polyetherfreien Formulierungen, die möglichst wenige Komponenten enthalten, die ausgehend von petrochemischen, nicht nachwachsenden Rohstoffen hergestellt werden. Daher besteht ein wichtiges Ziel der Kosmetikforschung darin, auf polyetherhaltige Inhaltsstoffe zu verzichten.
Jedoch zeigen polyetherfreie Produkte nach dem Stand der Technik nicht die gewünschten Eigenschaftsprofile. Polyetherfreie Formulierungen weisen z.B. deutlich reduzierte Schaumeigenschaften auf, was als deutlicher Nachteil angesehen wird. Ferner sind polyetherfreie tensidische Formulierungen deutlich schwieriger zu verdicken, da NaCl keine verdickenden Eigenschaften in solchen Systemen hat. Der Formulierer ist daher gezwungen auf die Klasse der assoziativen, hochethoxylierten Fettderivate zurückzugreifen und gibt dabei das Ziel der polyetherfreien Formulierungen auf.
Es gilt folglich traditionelle Wege zu verlassen und neue polyetherfreie Formulierungen zu entwickeln, die die konventionellen polyetherhaltigen Formulierungen ersetzen, sowie die modernen Anforderungen der Verbraucher erfüllen. Hierfür werden neue polyetherfreie Wirkstoffe mit sehr guten Applikationseigenschaften benötigt.

Sorbitol ist die reduzierte Polyolform der Glucose, zählt zu den Zuckeralkoholen und ist auch unter den Namen Sorbit oder Glucitol bekannt.

Sorbitol kann unter Abspaltung von Wasser eigenkondensieren, hierbei entsteht das sogenannte Sorbitan. Unter Sorbitan versteht man im Allgemeinen ein Produktgemisch der Eigenkondensationsprodukte des Sorbitols; diese sind im Wesentlichen fünf- und sechsgliederige, mono- und bicyclische, hydroxyfunktionelle Ether polyolischen Charakters, wie exemplarisch durch die folgenden Formeln dargestellt:

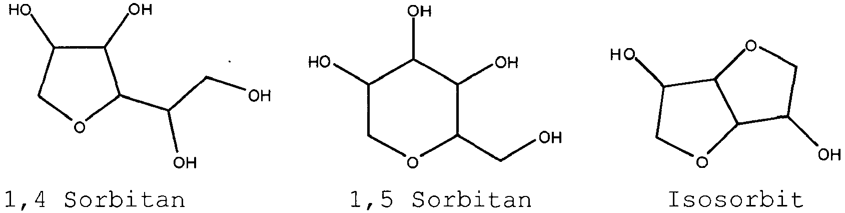

In diesem Gemisch sind im Allgemeinen im untergeordneten Ausmaß weitere Kondensationsprodukte und auch Sorbitol enthalten.

Sorbitanester sind die Ester des Sorbitans und somit die Veresterungsprodukte dieses oben beschriebenen Polyolgemisches mit organischen Säuren, wobei das Polyolgemisch in der Regel mit 1 bis 3 mol Säure pro mol Polyolgemisch verestert ist.

Eine Übersichtsdarstellung von Sorbitanestern findet sich beispielsweise in Treon, Soap Perfumary Cosmetics, January 1965, p. 47.

Sorbitanester sind als gute und milde Emulgatoren seit langem bekannt, jedoch haben nur längerkettige Fettsäurederivate von Mono-Laurat bis zum Tri-Stearat bisher eine industrielle Bedeutung. Traditionelle Sorbitanester sind zunächst nicht wasserlöslich. Sie werden daher bei Bedarf durch Ethoxylierung hydrophiliert. Für die Emulgierarbeit wird dann durch Mischen der hydrophilen und hydrophoben Sorbitanester der gewünschte HLB-Wert eingestellt und somit die Löslichkeit in den diversen Systemen ermöglicht.

DE 10 2004 036 067 beschreibt die Verwendung von Sorbitanestern als Reinigungsverstärker in wässrigen Reinigungsmittelkonzentraten auf Basis von nichtionischen, anionischen oder amphoteren Tensiden und gegebenenfalls unter Mitverwendung von üblichen Hilfs- und Zusatzstoffen zur Reinigung von profilierten Fliesen und Platten.

EP 1813251 beschreibt unter anderem die Verwendung von Sorbitanpartialestern zur Herstellung von polyetherfreien, kaltherstellbaren langzeitstabilen, niedrigviskosen und feinzelligen Öl-in-Wasser-Emulsionen für Feuchttücher (Wet Wipes).

US 7135168 beschreibt die Verwendung von Sorbitanestern und ethoxylierten Sorbitanestern in Haarfärbemitteln.

EP0843713 beschreibt milde Haarshampoos enthaltend langkettige Fettsäure-N-alkylglucamide und Zuckertenside. Unter anderem kommen hier als Zuckertenside, die die Anwendungseigenschaften der Fettsäure-N-alkylglucamide verbessern sollen, auch Sorbitanester, bevorzugt Sorbitanester von C₁₂- bis C₁₈-Carbonsäuren, in Frage. Fettsäure-N-alkylglucamide sind milde polyetherfreie Tenside, die jedoch auf Grund ihrer aufwändigen Herstellung keine verbreitete Anwendung gefunden haben.

Die EP1394225 beschreibt Wasser-in-Öl-Verdickerdispersionen, bei denen der Verdickungseffekt auf Polyelektrolyten beruht, und Sorbitanester von C₁₀- bis C₂₀-Carbonsäuren als Dispergatorkomponente eingesetzt werden.

Keine der genannten Schriften offenbart also Formulierungen zur Reinigung und Pflege von menschlichen oder tierischen Körperteilen enthaltend Sorbitanester so wie die Verwendung von Sorbitanestern als Viskositätsregulator, Pflegewirkstoff, Schaum-Booster oder Solubilisator.

Aufgabe der vorliegenden Erfindung ist es, Formulierungen bereitzustellen, die mit geringen Mengen an herkömmlichen Verdickern bzw. Viskositätsregulatoren auskommen und dabei eine gute Pflegeleistung aufweisen.

### Beschreibung der Erfindung

Überraschenderweise wurde gefunden, dass die im Folgenden beschriebenen Formulierungen zur Reinigung und Pflege von menschlichen oder tierischen Körperteilen so wie die erfindungsgemäße Verwendung von Umsetzungsprodukten des Sorbitols diese Aufgabe lösen.

Gegenstand der vorliegenden Erfindung sind daher Formulierungen zur Reinigung und Pflege von menschlichen oder tierischen Körperteilen enthaltend Verfahrensprodukte von Veresterungsreaktionen des Sorbitols.
Ein weiterer Gegenstand der Erfindung ist die Verwendung von Verfahrensprodukten von Veresterungsreaktionen des Sorbitols als Viskositätsregler, Pflegewirkstoff, Schaum-Booster oder Solubilisator.

Ein Vorteil der Erfindung ist die gute Verfügbarkeit der eingesetzten Komponenten sowie deren, besonders für kosmetische und pharmazeutische Anwendungen relevanten, gute toxikologische Eigenschaften.
Ein weiterer Vorteil ist, dass aufgrund der hohen Anwendungsleistung auf weitere Viskositätsregulatoren und gegebenenfalls auch auf weitere Rückfetter, Schaum-Booster oder Solubilisatoren in der jeweils gewählten Formulierung verzichtet werden kann.
Noch ein weiterer Vorteil ist, dass die erfindungsgemäße Verwendung auch in polyetherfreien tensidischen Formulierungen als Viskositätsregler, Pflegewirkstoff, Schaum-Booster oder Solubilisator möglich ist.
Wie oben begründet, versagen viele konventionelle Verdicker wie beispielsweise NaCl in polyetherfreien Formulierungen und die hochmolekularen, assoziativen Verdicker können wegen ihrer Polyethergruppen in polyetherfreien Formulierungen nicht eingesetzt werden.
Ferner sind die Schaumeigenschaften von PEG-freien und polyetherfreien Systemen nicht ausreichend.
Alle angegebenen Prozentangaben sind, wenn nicht anders angegebenen, Gewichtsprozentangaben.
Gegenstand der vorliegenden Erfindung sind somit Formulierungen zur Reinigung und Pflege von menschlichen oder tierischen Körperteilen enthaltend Sorbitancarbonsäureester, dadurch gekennzeichnet, dass der Carbonsäureanteil des Sorbitancarbonsäureesters sich ableitet von einer Carbonsäure enthaltend 6 bis 10 Kohlestoff-Atome und die Sorbitancarbonsäureester eine Hydroxylzahl (OH-Zahl) von größer 350, bevorzugt von größer 400, insbesondere von größer 450 aufweisen, dadurch gekennzeichnet , dass die Formulierung im Wesentlichen frei von Polyethern und Polyether enthaltenden Verbindungen ist und dass 1,2 bis 2 Mol Carbonsäure pro Mol Sorbitol, von dem sich der Sorbitancarbonsäureester ableitet, eingeestert sind. Geeignete Bestimmungsmethoden zur Ermittlung der Hydroxylzahl sind insbesondere solche gemäß DGF C-V 17 a (53), Ph. Eur. 2.5.3 Method A und DIN 53240.
Bevorzugt ist, dass die erfindungsgemäße Formulierung von 0,01 Gew.-% bis 10 Gew.-%, bevorzugt 0,05 Gew.-% bis 5 Gew.-%, besonders bevorzugt 0,1 Gew.-% bis 3 Gew.-% Sorbitancarbonsäureester bezogen auf die Gesamtformulierung enthält.

Bevorzugt handelt es sich bei den erfindungsgemäßen Formulierungen um wässrige Formulierungen; solche sind im Sinne der vorliegenden Erfindung gekennzeichnet durch einen Wassergehalt von mindestens 50 Gew.-%, bevorzugt mindestens 75 Gew.-%, besonders bevorzugt mindestens 80 Gew.-% bezogen auf die Gesamtformulierung.

Erfindungsgemäße Formulierungen sind tensidische Formulierungen sind. Da die in erfindungsgemäßen Formulierungen enthaltenen Sorbitancarbonsäureester bereits als beispielsweise Sorbitancaprylat enthaltende Zusammensetzung tensidische Eigenschaften besitzt, ist es im Zusammenhang mit der vorliegenden Erfindung unter dem Begriff "tensidische Formulierung" eine Formulierung zu verstehen, die neben dem Sorbitancarbonsäureester mindestens ein weiteres Tensid enthält. Enthaltene weitere Tenside können beispielsweise nichtionische, anionische oder amphotere Tenside sein. Typische Beispiele für milde, d. h. besonders hautverträgliche Tenside sind polyetherfrei, z.B. Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen. Amphotere Tenside sind z.B. Betaine, Amphoacetate oder Amphopropionate einzusetzen, so z.B. Substanzen wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ampholytischen Tensiden sind z.B. solche oberflächenaktiven Verbindungen die außer einer C8/18-Alkyl- oder -Acylgruppe im Molekül mindestens eine freie Aminogruppe und mindestens eine -COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-Kokosalkylaminopropionat, das Kokosacylaminoethylaminopropionat und das C12/18-Acylsarcosin.
Erfindungsgemäße tensidische Formulierungen enthalten als weiteres Tensid insbesondere Fettalkoholsulfate, Mono- und/oder Dialkylsulfosuccinate, Amphoacetate, Amphopropionate, Betaine, Cocamidopropylbetaine, Alkyloligoglucoside oder Fettsäureglutamate.

Die erfindungsgemäßen tensidischen Formulierungen enthalten als weiteres Tensid solche Tenside, welche im Wesentlichen frei von Polyethern und Polyether enthaltenden Verbindungen sind.

Besonders bevorzugt enthalten erfindungsgemäße tensidische Formulierungen als weitere Tenside die polyetherfreien Tenside Mono- und/oder Dialkylsulfosuccinate, Amphoacetate, Amphopropionate, Betaine, insbesondere Cocamidopropylbetaine, Alkyloligoglucoside oder Fettsäureglutamate.

Bevorzugte erfindungsgemäße tensidische Formulierungen enthalten mindestens 2 Gew.-%, bevorzugt mindestens 4 Gew.-% und besonders bevorzugt mindestens 6 Gew.-% mindestens eines weiteren Tensids bezogen auf die Gesamtformulierung.

Besonders bevorzugte erfindungsgemäße Formulierungen sind wässrige tensidische Formulierungen enthaltend mindestens 0,01 Gew.-% Sorbitancarbonsäureester, mindestens 5 Gew.-% mindestens eines weiteren Tensids und mindestens 75 Gew.-% Wasser bezogen auf die Gesamtformulierung, insbesondere solche enthaltend mindestens 0,3 Gew.-% Sorbitancarbonsäureester, mindestens 6 Gew.-% mindestens eines weiteren Tensids und mindestens 80 Gew.-% Wasser bezogen auf die Gesamtformulierung.

Bevorzugt stellen erfindungsgemäße Formulierungen flüssige, kosmetische, dermatologische oder pharmazeutische Körperreinigungsmittel, insbesondere Duschbäder und -gele, Badeformulierungen, Flüssigseifen und Shampoos dar.
Für bevorzugte erfindungsgemäße Formulierungen gilt somit, dass die menschlichen oder tierischen Körperteile bevorzugt Haare oder Haut sind.

Ebenso sind erfindungsgemäße Formulierungen bevorzugt, die dadurch gekennzeichnet sind, dass die Formulierung frei von Fettsäure-N-alkylglucamiden ist.

Der Begriff "im Wesentlichen frei von Polyethern und Polyether enthaltenden Verbindungen" beschreibt im Zusammenhang mit der vorliegenden Erfindung, dass enthaltene Verbindungen nur in Spuren, bevorzugt keine, Alkoxygruppen, Oligoalkoxygruppen oder Polyalkoxygruppen wie z.B. Ethylenoxid oder Propylenoxid enthalten. Die Konzentration an Polyether enthaltenden Verbindungen sollte kleiner 0,1 Gew.-%, insbesondere bevorzugt kleiner 0,01 Gew.-% bezogen auf die Gesamtformulierung, bevorzugt unterhalb der Nachweisgrenze gängiger Analyseverfahren wie beispielsweise NMR-Spektroskopie, GPC oder Maldi sein. Analoges gilt für den Begriff "frei von Fettsäure-N-alkylglucamiden".

Aufgrund der guten Verfügbarkeit und der einfachen Handhabung enthalten erfindungsgemäße Formulierungen bevorzugt Sorbitancarbonsäureester, bei denen der Carbonsäureanteil sich ableitet von Ethylhexansäure oder Caprylsäure, bevorzugt Caprylsäure.
Es ist offenbar, dass es sich im Zusammenhang mit der Erfindung anbietet, dass erfindungsgemäße Formulierungen bevorzugt Sorbitancarbonsäureester, bei denen der Carbonsäureanteil sich ableitet von technischer Caprylsäure, insbesondere von technischer Caprylsäure aus nativen Rohstoffen, wie sie z.B aus Palm- oder Kokosöl, gewonnen wird.
Diese technische Caprylsäure kann Carbonsäuren mit einer Kettenlänge von 6 bis 10 Kohlenstoffatomen umfassen, wobei die wesentliche Komponente durch Carbonsäuren mit einer Kettenlänge von 8 Kohlenstoffatomen gebildet wird: Unter "wesentlicher Komponente" sind mindestens 50 Gew.-%, bevorzugt 80 Gew.-%, insbesondere 90 Gew.-% bezogen auf das Gesamtgewicht der technischen Säuremischung zu verstehen.

Erfindungsgemäße Formulierungen enthalten besonders bevorzugt Sorbitancarbonsäureester, die dadurch gekennzeichnet sind, dass sie eine Säurezahl von kleiner 20, bevorzugt von kleiner 15, insbesondere von kleiner 10 aufweisen. Geeignete Bestimmungsmethoden zur Ermittlung der Säurezahl sind insbesondere solche gemäß DGF C-V 2, Ph.Eur. 2.5.1, ISO 3682, ASTM D 974 und DIN EN ISO 2114.

Erfindungsgemäße Formulierungen enthalten besonders bevorzugt Sorbitancarbonsäureester, die dadurch gekennzeichnet sind, dass sie eine Iodzahl von kleiner 30, bevorzugt von kleiner 10, insbesondere von kleiner 5 aufweisen.

Geeignete Bestimmungsmethoden zur Ermittlung der Iodzahl sind insbesondere solche gemäß DGF C-V 11 a (53), Ph. Eur. 2.5.4 Method A und DIN 53241.

Erfindungsgemäße Formulierungen enthalten besonders bevorzugt Sorbitancarbonsäureester, die dadurch gekennzeichnet, dass sie eine Viskosität von 100 bis 20000 mPa·s, bevorzugt von 1000 bis 15000 mPa·s, insbesondere von 2000 bis 10000 mPa·s aufweisen.
Geeignete Bestimmungsmethode zur Ermittlung der Viskosität ist insbesondere die gemäß DIN 53015.

Erfindungsgemäße Formulierungen enthalten besonders bevorzugt Sorbitancarbonsäureester, bei denen 1 bis 3 Mol, bevorzugt 1,2 bis 2 Mol, insbesondere 1,4 bis 1,6 Mol Carbonsäure pro einem Mol Sorbitol, von dem sich der Sorbitancarbonsäureester ableitet, eingeestert sind.

Erfindungsgemäße Formulierungen enthalten besonders bevorzugt Sorbitancarbonsäureester kommerziell erhältlich (Evonik Industries) unter INCI Namen Sorbitan Sesquicaprylate.

Die Herstellung der in erfindungsgemäßen Formulierungen enthaltenen Sorbitancarbonsäureester kann durch Kondensation und Ver- oder Umesterungsreaktionen von Sorbitol mit Carbonsäuren oder deren Ester erfolgen. Bei dieser Herstellung können je nach Ausprägung des Verfahrens die Reaktionen nacheinander oder parallel ab. Erfindungsgemäße Formulierungen enthalten insbesondere Sorbitancarbonsäureester erhältlich durch, bevorzugt erhalten durch ein Verfahren umfassend die Verfahrensschritte A) Dehydratisierung von Sorbitol, B) Umsetzung des dehydratisierten Sorbitols mit mindestens einer Verbindung ausgewählt aus der Gruppe umfassend Carbonsäuren enthaltend 6 bis 10, bevorzugt 8, Kohlenstoffatome, Carbonsäureester oder Carbonsäureestermischungen, bei denen die Carbonsäurekomponente 6 bis 10, bevorzugt 8, Kohlenstoffatome enthält, und gegebenenfalls C) Isolierung von gebildetem Sorbitanester aus Verfahrensschritt B).

In Verfahrensschritt A) wird das Sorbitol zu verschiedenen Isomeren dehydratisiert, so beispielsweise zu 1,4- und 3,6-Sorbitan. Die Reaktionsbedingungen in Verfahrensschritt A) haben einen Einfluss auf die Zusammensetzung des Dehydratisierungsproduktes.
Erfindungsgemäße Formulierungen zeichnen sich dadurch aus, dass bevorzugt Sorbitancarbonsäureester enthalten sind, bei deren Herstellung Verfahrensschritt A) bei einer Temperatur zwischen 100 °C bis 180 °C, bevorzugt zwischen 120 °C bis 160 °C, insbesondere zwischen 130 °C bis 150 °C durchgeführt wird.
Des weiteren zeichnen sich erfindungsgemäße Formulierungen dadurch aus, dass bevorzugt Sorbitancarbonsäureester enthalten sind, bei deren Herstellung Verfahrensschritt A) bei einem Druck zwischen 0,001 bar bis 1,5 bar, bevorzugt zwischen 0,5 bar bis 1,25 bar, insbesondere zwischen 0,8 bar bis 1,2 bar durchgeführt wird.

In einer bevorzugten, alternativen Ausführungsform erfindungsgemäßer Formulierungen werden
Sorbitancarbonsäureester eingesetzt, bei deren Herstellung Verfahrensschritt A) bei einem Druck zwischen 0,001 bar bis 0,9 bar, bevorzugt zwischen 0,005 bar bis 0,5 bar, insbesondere zwischen 0,006 bar bis 0,01 bar und bei einer Temperatur zwischen 80 °C bis 140 °C, bevorzugt zwischen 90 °C bis 130 °C, insbesondere zwischen 95 °C bis 120 °C durchgeführt wird.

Der Einsatz eines sauren Katalysators wie beispielsweise in EP 0280780 beschrieben kann einen Einfluss auf das Dehydratisierungsprodukt haben. Erfindungsgemäße Formulierungen enthalten bevorzugt Sorbitancarbonsäureester, bei denen Verfahrensschritt A) mit einem sauren Katalysator, bevorzugt Phosphorsäure, durchgeführt wird.

Eine schnelle und möglichst quantitative Umsetzung in Verfahrensschritt B) ist abhängig von verschiedenen Parametern wie Druck, Temperatur und Mengenverhältnis der Reaktionspartner zueinander. Diese Parameter beeinflussen ebenfalls die Sorbitancarbonsäureester hinsichtlich statistischer Verteilung, beispielsweise von verschiedenen Isomeren, hervorgerufen durch z.B. unterschiedliche Möglichkeiten der Veresterungsposition im Molekül, die zu Gemischen von Mono-, Di- und sogar Triestern führen können.

Erfindungsgemäße Formulierungen enthalten bevorzugt Sorbitancarbonsäureester, die dadurch gekennzeichnet sind, dass Verfahrensschritt B) bei einer Temperatur zwischen 140 °C bis 260 °C, bevorzugt zwischen 160 °C bis 250 °C, insbesondere zwischen 200 °C bis 230 °C durchgeführt wird. Analog ist es bevorzugt, dass Verfahrensschritt B) bei einem Druck zwischen 0,001 bar bis 1,5 bar, bevorzugt zwischen 0,5 bar bis 1,25 bar, insbesondere zwischen 0,8 bar bis 1,2 bar durchgeführt wird.

In einer bevorzugten, alternativen Ausführungsform erfindungsgemäßer Formulierungen werden
Sorbitancarbonsäureester eingesetzt, bei deren Herstellung Verfahrensschritt B) bei einem Druck zwischen 0,001 bar bis 0,9 bar, bevorzugt zwischen 0,05 bar bis 0,5 bar, insbesondere zwischen 0,006 bar bis 0,01 bar und bei einer Temperatur zwischen 80 °C bis 250 °C, bevorzugt zwischen 120 °C bis 220 °C, insbesondere zwischen 150 °C bis 200 °C durchgeführt wird.

Ebenso wie in Verfahrensschritt A) kann der Einsatz eines Katalysators in Verfahrensschritt B) wie beispielsweise Alkalihydroxiden, Alkalimetallcarbonaten oder Alkalisalze von Phosphorsäure, Phosphoriger Säure oder Unterphosphoriger Säure einen Einfluss auf die Sorbitancarbonsäureester haben.
Erfindungsgemäße Formulierungen enthalten bevorzugt Sorbitancarbonsäureester, bei deren Herstellung in Verfahrensschritt B) mindestens ein Katalysator ausgewählt aus der Gruppe umfassend Alkalimetallsalze und Erdalkalimetallsalze, bevorzugt Natriumhydroxid, eingesetzt wird.

Es ist offenbar, dass es sich im Zusammenhang mit der Erfindung anbietet, technische Caprylsäure, insbesondere technische Caprylsäure aus nativen Rohstoffen, wie sie z.B aus Palm- oder Kokosöl, gewonnen wird , in Verfahrensschritt B) als Carbonsäure einzusetzen. Bezüglich der Zusammensetzung der technischen Caprylsäure, siehe oben.

Die erfindungsgemäße Formulierungen können Sorbitancarbonsäureester enthalten, bei denen in Verfahrensschritt B) Carbonsäureester jeglicher Carbonsäure enthaltend 8 Kohlenstoffatome eingesetzt werden; es ist in diesem Zusammenhang allerdings bevorzugt, dass der eingesetzte Carbonsäureester ein Ester von einer Carbonsäure enthaltend 8 Kohlenstoffatome mit mindestens einem Alkohol ausgewählt aus der Gruppe umfassend Glycerin, Methanol und Ethanol ist.

In zu oben analoger Weise ist es bevorzugt, dass der eingesetzte Carbonsäureester bevorzugt ein Ester der Ethylhexansäure oder der Caprylsäure ist, wobei die Caprylsäure sich wie oben beschrieben insbesondere von technischer Caprylsäure, insbesondere von technischer Caprylsäure aus nativen Rohstoffen, ableitet.
Insbesondere lassen sich auch Carbonsäureester aus nativen Rohstoffen, wie z.B. Glycerinester der Caprylsäure aus Ziegenbutter, Milch, Palm- und Kokosöl oder aus Weinfuselöl als Carbonsäureester in Verfahrensschritt B) einsetzen.

Dem Fachmann ist ersichtlich, dass sich ebenso Mischungen verschiedener Edukte in den unterschiedlichen Verfahrensschritten einsetzen lassen können, so wie in den erfindungsgemäßen Formulierungen Mischungen von Sorbitancarbonsäureester enthalten sein können.

Es ist offenbar, dass sich die Eigenschaften der erfindungsgemäßen Formulierungen durch das im Verfahren eingesetzte Mengenverhältnis von Carbonsäure bzw. Carbonsäureester zu Sorbitol beeinflussen lassen. Erfindungsgemäße Formulierungen zeichnen sich dadurch aus, dass bevorzugt Sorbitancarbonsäureester enthalten sind, bei deren Herstellung das Mol-Verhältnis von in Verfahrensschritt A) eingesetztem Sorbitol zu in Verfahrensschritt B) eingesetztem Umsetzungspartner zwischen 1:1 bis 1:3, bevorzugt zwischen 1:1,2 bis 1:2, insbesondere zwischen 1:1,4 bis 1:1,6 liegt.

Da es aus prozesstechnischer Sicht vorteilhaft sein kann, wenn Verfahrensschritt A) und B) durch von Außen leicht beeinflussbare Parameter eingeleitet werden, enthalten erfindungsgemäße Formulierungen bevorzugt Sorbitancarbonsäureester, die dadurch gekennzeichnet sind, dass alle in Verfahrensschritt A) und B) eingesetzten Substanzen zu Beginn von Verfahrensschritt A) enthalten sind; bevorzugt in diesem Zusammenhang wird Verfahrensschritt B) durch eine dynamische Temperaturerhöhung initiiert.

In Verfahrensschritt C) kann eine Isolierung von gebildetem Sorbitanester durchgeführt werden.
Zur Isolierung der Sorbitanester kommen alle dem Fachmann bekannten Methoden zur Isolierung von niedermolekularen Substanzen aus komplexen Zusammensetzungen in Betracht. Beispielhaft seien an dieser Stelle die Fällung mittels geeigneter Lösungsmittel, die Extraktion mittels geeigneter Lösungsmittel, die Komplexierung, beispielsweise mittels Cyclodextrinen oder Cyclodextrin-Derivaten, die Kristallisation, die Aufreinigung bzw. Isolierung mittels chromatographischer Methoden oder die Überführung der Sorbitanester in leicht abtrennbare Derivate genannt. Erfindungsgemäße Formulierungen enthalten bevorzugt Sorbitancarbonsäureester, die ohne Verfahrensschritt C) erhalten wurden.

Die erfindungsgemäßen Pflegeformulierungen können z.B. mindestens eine zusätzliche Komponente enthalten, ausgewählt aus der Gruppe der
Emollients,
Emulgatoren,
Verdicker/Viskositätsregler/Stabilisatoren, UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe, Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden.

Ein weiterer Gegenstand der vorliegenden Erfindung ist die Verwendung von Sorbitancarbonsäureestern, bei denen der Carbonsäureanteil sich ableitet von einer Carbonsäure enthaltend 6 bis 10 Kohlestoff-Atome und die eine Hydroxylzahl (OH-Zahl) von größer 350, bevorzugt von größer 400, insbesondere von größer 450 aufweisen, als Viskositätsregler, Pflegewirkstoff, Schaum-Booster oder Solubilisator in polyetherfreien tensidischen Formulierungen, dadurch gekennzeichnet, dass 1,2 bis 2 Mol Carbonsäure pro Mol Sorbitol, von dem sich der Sorbitancarbonsäureester ableitet, eingeestert sind.

Die reinigenden oder pflegenden Formulierungen, in denen die Sorbitancarbonsäureester erfindungsgemäß verwendet werden, sind bevorzugt kosmetische, dermatologische oder pharmazeutische Formulierungen, bevorzugt zur Reinigung und Pflege von menschlichen oder tierischen Körperteilen, besonders bevorzugt tensidische, insbesondere wässrige tensidische Formulierungen zur Reinigung und Pflege von menschlichen oder tierischen Körperteilen, insbesondere von Haut und Haar.
Weitere bevorzugte Formulierungen, in denen die Sorbitancarbonsäureester erfindungsgemäß verwendet werden, sind oben beschriebene, bevorzugte erfindungsgemäße Formulierungen.
Bevorzugt werden bei erfindungsgemäßer Verwendung Sorbitancarbonsäureester verwendet, die im Rahmen der oben beschriebenen erfindungsgemäßen Formulierungen in den erfindungsgemäßen Formulierungen bevorzugt enthalten sind. In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

Alle Konzentrationen in den Anwendungsbeispielen sind in Gewichtsprozent angegeben. Zur Herstellung der Formulierungen wurden dem Fachmann bekannte übliche Formulierungsverfahren eingesetzt.

### Beispiel 0: Herstellung Sorbitancarbonsäureester

390,45g Sorbitol Sirup, eine 70%ige wässrige Lösung, 2,9g Phosphorsäure und 5,0g Natriumhydroxid wurden in einem Kolben eingewogen und bei Atmosphärendruck und bei 140°C für 30 min dehydratisiert. Anschließend wurden 334,8g Caprylsäure zugegeben und bei 200°C und Atmosphärendruck verestert. Nach der Reaktionszeit wurde das Produkt über eine Filterpresse filtriert. Das erhaltene Produkt ist klar, besitzt eine Endviskosität von ca. 6000 mPa·s, eine Hydroxylzahl von 470, eine Säurezahl von < 10 und eine Iodzahl von < 1, bestimmt nach den oben beschriebenen Verfahren.

Die erhaltene Substanz wurde in den folgenden Beispielen eingesetzt und wird im Folgenden mit Cap01 bezeichnet

### Beispiel 1: Austestung der Verdickungseigenschaften

Die verdickende Wirkung des Cap01 aus Beispiel 0 wurde im Vergleich zu gängigen tensidischen Verdickern in verschiedenen Tensidsystemen getestet.

Die Viskosität wurden mittels eines Brookfield Viskosimeters (Brookfield LVF, Spindel 3, 5 Upm) bei 25 °C gemessen.

### 1a) Tensidsystem la (nicht erfindungsgemäß):

32 Gew.-% Natriumlaurylethersulfat (Cognis, Texapon^{®} NSO, 28%-ig), 8 Gew.-% Cocamidopropylbetain (Evonik Goldschmidt GmbH, TEGO^{®} Betain F 50, 38%-ig) und 0,7 Gew.-% NaCl, wurde auf eine Viskosität von 3500 mPas bei 25 °C eingestellt. Die dazu jeweils benötigte Verdickerkonzentration ist in Tabelle 1-1 dargestellt. Es zeigt sich, dass Cap01 aus Beispiel 0 im Vergleich zu marktüblichen Verdickern am effektivsten ist, da die geringste Einsatzkonzentration benötigt wird.

**Tabelle 1-1: Verdickungswirkung von Cap01 im Vergleich zu marktüblichen Verdickern.**

| | Gew.-% | Gew.-% | Gew.-% |
|---|---|---|---|
| Texapon NSO^{®} (Cognis, INCI: Sodium Laureth Sulfate, 28 %-ig) | 32,0 | 32,0 | 32,0 |
| TEGO^{®} Betain F 50 (Evonik Goldschmidt GmbH, INCI: Cocamidopropyl Betaine, 38 %-ig) | 8,0 | 8,0 | 8, 0 |
| Cap01 | 1,1 | | |
| REWOMID^{®} DC 212 S (Evonik Goldschmidt GmbH, INCI: Cocamide DEA) * | | 1,5 | |
| Tegosoft^{®} PC 31 (Evonik Goldschmidt GmbH, INCI: Polyglyceryl-3 Caprate) * | | | 2,4 |
| NaCl | 0,7 | 0,7 | 0,7 |
| Wasser, demineralisiert | ad 100,0 | | |
| Viskosität [mPas], | 3500 | | |

| | | | |
|---|---|---|---|
| * Vergleichsbeispiel nicht erfindungsgemäß | | | |

### 1b) Tensidsystem 1b (nicht erfindungsgemäß):

32 Gew.-% Natriumlaurylethersulfat (Cognis, Texapon^{®} NSO, 28 %-ig) und 9 Gew.-% Sodium Cocoamphoacetate (Evonik Goldschmidt GmbH, Rewoteric^{®} AM C, 32 %-ig) wurde auf eine Viskosität von 3500 mPas bei 25 °C eingestellt. Die dazu jeweils benötigte Verdickerkonzentration ist in Tabelle 1-2 dargestellt. Es zeigt sich, dass Cap01 im Vergleich zu marktüblichen Verdickern am effektivsten ist, da die geringste Einsatzkonzentration benötigt wird.

**Tabelle 1-2: Verdickungswirkung von Cap01 im Vergleich zu marktüblichen Verdickern.**

| | Gew.-% | Gew.-% | Gew.-% |
|---|---|---|---|
| Texapon^{®} NSO (Cognis, INCI: Sodium Laureth Sulfate, 28 %-ig) | 32,0 | 32,0 | 32,0 |
| Rewoteric^{®} AM C (Evonik Goldschmidt GmbH, INCI: Sodium Cocoamphoacetate, 32 %-ig) | 9,0 | 9,0 | 9,0 |
| Cap01 | 1,5 | | |
| REWOMID^{®} DC 212 S (Evonik Goldschmidt GmbH, INCI: Cocamide DEA) * | | 1,9 | |
| Tegosoft^{®} PC 31 (Evonik Goldschmidt GmbH, INCI: Polyglyceryl-3 Caprate)* | | | 3.9 |
| Wasser, demineralisiert | ad 100,0 | | |
| Viskosität [mPas], | 3500 | | |

| | | | |
|---|---|---|---|
| * Vergleichsbeispiel nicht erfindungsgemäß | | | |

### 1c) Tensidsystem 1c:

15 Gew.-% Sodium Cocoamphoacetate (Evonik Goldschmidt GmbH, Rewoteric^{®} AM C, 32 %-ig), 13 Gew.-% Cocamidopropylbetain (Evonik Goldschmidt GmbH, TEGO^{®} Betain F 50, 38 %-ig) und 3,8 Gew.-% Disodium Lauryl Sulfosuccinate (Evonik Goldschmidt GmbH, Rewopol^{®} SB F 12 P, 95 %-ig) wurde auf eine Viskosität von 3500 mPas bei 25 °C eingestellt.
Hierbei handelt es sich um eine PEG-freie Tensidformulierung, die schwer zu verdicken ist.
In Tab. 1-3 wird dargestellt, welche Einsatzkonzentration des marktüblichen Verdickers ANTIL^{®} HS 60 (Evonik Goldschmidt GmbH, INCI: Cocamidopropyl Betaine; Glyceryl Laurate) im Vergleich zu der Kombination Cap01 + ANTIL^{®} HS 60 (Evonik Goldschmidt GmbH, INCI: Cocamidopropyl Betaine; Glyceryl Laurate) benötigt wird. Es wird offensichtlich, dass eine synergistische Wirkung bei der Verwendung der Kombination Cap01 + ANTIL^{®} HS 60 (Evonik Goldschmidt GmbH, INCI: Cocamidopropyl Betaine; Glyceryl Laurate) auftritt und eine signifikante Reduzierung der benötigten Verdickermenge von 4,5 Gew.-% auf 1,5 Gew.-% erzielt werden kann. Auch hier kommt es zu einer Resourcenschonung, da deutlich weniger Verdickerwirkstoffe benötigt werden.

**Tabelle 1-3: Verdickungswirkung von Cap01 in einer PEG-freien Formulierung im Vergleich zu marktüblichen Verdickern.**

| | Gew.-% | |
|---|---|---|
| Rewopol^{®} SB F 12 P (Evonik Goldschmidt GmbH, INCI: Disodium Lauryl Sulfosuccinate, 95 %-ig) | 3,8 | 3,8 |
| TEGO^{®} Betain F 50 (Evonik Goldschmidt GmbH, INCI: Cocamidopropyl Betaine, 38 %-ig) | 13 | 13 |
| Rewoteric^{®} AM C (Evonik Goldschmidt GmbH, INCI: Sodium Cocoamphoacetate, 32 %-ig) | 15 | 15 |
| Cap01 | 0 | 0,5 |
| Antil HS 60 | 4,5 | 1 |
| Wasser, demineralisiert | ad 100,0 | |
| Viskosität [mPas], | 3500 | |

### ld) Verwendung von Cap01 als Verdicker in reinigender Formulierung für harte Oberflächer (nicht erfindungsgemäß)

| %-Aktivgehalte | Gel-1 | Gel-2 |
|---|---|---|
| Texapon^{®} NSO (Cognis, INCI: Sodium Laureth Sulfate, 28 %-ig) | 48, 6 | 48, 6 |
| TEGO^{®} Betain F 50 (Evonik Goldschmidt GmbH, INCI: Cocamidopropyl Betaine, 38 %-ig) | 9,0 | |
| Rewoteric^{®} AM C (Evonik Goldschmidt GmbH, INCI: Sodium Cocoamphoacetate, 32 %-ig) | | 10, 6 |
| NaCl | 0,7 | |
| Cap01 | 1,8 | 1,8 |
| Wasser | | |
| Aussehen | Klar | Klar |
| pH-Wert | | |
| Viskosität [mPas] | 23000 | 5900 |

Beispiel 2: Austestung der Konditionierung von Haut

### (Hautpflegeleistung) und Schaumeigenschaften mittels eines Handwaschtests

Zur Bewertung der rückfettenden Pflege von Haut (Hautpflegeleistung) und der Schaumeigenschaften von Cap01 in wässrigen, tensidischen Formulierungen wurden sensorische Handwaschtests im Vergleich zu dem Marktstandard Polyethylenglykol(7)glycerylmonoacetat durchgeführt.

Polyethylenglykol(7)glycerylmonococoat ist in der Industrie als rückfettender Pflegewirkstoff weit verbreitet und gilt als hochwirksame Komponente in wässrigen, tensidischen Formulierungen.

Eine Gruppe bestehend aus 10 trainierten Prüfpersonen wusch sich dabei definiert die Hände und bewertete Schaumeingeschaften und Hautgefühl anhand einer Notenskala von 1 (schlecht) bis 5 (sehr gut). Die eingesetzten Produkte wurden jeweils in einer standardisierten Tensidformulierung (Tabelle 2-1 und 2-3) getestet.

### 2a) Handwaschtest in einer konventionellen polyelherhaltigen (nicht erfindungsgemäß)

Als Kontrollformulierung 2a1 wird eine Formulierung ohne Zusatz eines Additivs verwendet.

**Tab.2-1: Testformulierungen für Handwaschtest.**

| **Formulierungs-Beispiele** | **2a1*** | **2a2** | **2a3*** |
|---|---|---|---|
| Texapon NSO^{®} (Cognis, INCI: Sodium Laureth Sulfate, 28 %-ig) | 32 % | 32 % | 32 % |
| TEGO^{®} Betain F 50 (Evonik Goldschmidt GmbH INCI: Cocamidopropyl Betaine, 38 %-ig) | 8 % | 8 % | 8 % |
| NaCl | 2 % | 2 % | 2 % |
| Wasser, demineralisiert | ad. 100 % | | |
| Cap01 | | 1.0 % | |
| Tegosoft^{®} GC (Evonik Goldschmidt GmbH, INCI: Polyethylenglykol(7)glycerylmonococoat) | | | 1.0 % |

| | | | |
|---|---|---|---|
| *Vergleichsbeispiel | | | |

Die Testergebnisse sind in der Tabelle 2-2 zusammengefaßt.

**Tab.2-2: Ergebnisse des Handwaschtests**

| **Testformulierung** | **2a1*** | **2a2** | **2a3*** |
|---|---|---|---|
| Anschäumverhalten | 3.1 | 3.7 | 3.1 |
| Schaumvolumen | 2.6 | 3.4 | 2.6 |
| Schaumcremigkeit | 2.6 | 3.4 | 2.6 |
| Hautgefühl während des Waschens | 3.0 | 4.0 | 3.6 |
| Hautglätte direkt nach der Applikation | 1.6 | 2.7 | 2.3 |
| Hautweichheit direkt nach der Applikation | 2.0 | 3.1 | 2.7 |
| Hautglätte nach 3 min. | 3.0 | 3.6 | 3.4 |
| Hautweichheit nach 3 min. | 3.0 | 3.5 | 3.3 |

| | | | |
|---|---|---|---|
| *Vergleichsbeispiel | | | |

In Tabelle 2-2 sind die Ergebnisse des Handwaschtests dargestellt. Anhand der Messergebnisse wird ersichtlich, dass die Formulierung 2a2 unter Verwendung von Cap01 eine bessere Hautglätte und Hautweichheit 3 Minuten nach der Applikation und ein überlegenes Hautgefühl während des Waschens im Vergleich zu den Vergleichsformulierungen 2a1 und 2a3 nach dem Stand der Technik bewirken. Auch die Hautglätte und Hautweichheit direkt nach der Applikation ist bei der erfindungsgemäßen Formulierungen 2a2 überlegen zu den Messwerten bei den Vergleichsformulierungen 2a1 und 2a3. Des Weiteren ist anhand der Messwerte ersichtlich, dass die Formulierung 2a2 mit Cap01 eine Verbesserung der Schaumeigenschaften bewirkt.

### 2b) Handwaschtest in einer polyetherfreien Tensidformulierung:

Als Kontrollformulierung 2a4 wird eine Formulierung ohne Zusatz eines Additivs verwendet.

**Tab.2-3: Testformulierungen für Handwaschtest.**

| **Formulierungs-Beispiele** | **2a4*** | **2a5** |
|---|---|---|
| Rewoteric^{®} AM C (Evonik Goldschmidt GmbH, INCI: Sodium Cocoamphoacetate, 32 %-ig) | 15 % | 15 % |
| TEGO^{®} Betain F 50 (Evonik Goldschmidt GmbH, INCI: Cocamidopropyl Betaine, 38 %-ig) | 13 % | 13 % |
| Rewopol^{®} SB F 12 P (Evonik Goldschmidt GmbH, INCI: Disodium Lauryl Sulfosuccinate 95%-ig) | 3.8 % | 3.8 % |
| Wasser, demineralisiert | ad 100 % | |
| Cap01 | | 0.5 % |

| | | |
|---|---|---|
| *Vergleichsbeispiel nicht erfindungsgemäß | | |

Die Testergebnisse sind in der Tabelle 2-4 zusammengefasst.

**Tab.2-4: Ergebnisse des Handwaschtests**

| **Testformulierung** | **2a4*** | **2a5** |
|---|---|---|
| Anschäumverhalten | 2.2 | 2.9 |
| Schaumvolumen | 1.7 | 2.3 |
| Schaumcremigkeit | 1.9 | 2.7 |
| Hautgefühl während des Waschens | 3.3 | 3.6 |
| Hautglätte direkt nach der Applikation | 2.3 | 2.8 |
| Hautweichheit direkt nach der Applikation | 2.7 | 2.9 |
| Hautglätte nach 3 min. | 3.2 | 3.5 |
| Hautweichheit nach 3 min. | 3.1 | 3.5 |

| | | |
|---|---|---|
| *Vergleichsbeispiel nicht erfindungsgemäß | | |

In Tabelle 2-4 sind die Ergebnisse des Handwaschtests dargestellt. Anhand der Messergebnisse wird ersichtlich, dass die erfindungsgemäße Formulierung 2a5 unter Verwendung von Cap01 ein überlegenes Hautgefühl während und nach des Waschens im Vergleich zu der Vergleichsformulierung 2a4 bewirken.

Des Weiteren ist anhand der Messwerte ersichtlich, dass die erfindungsgemäße Formulierung 2a5 mit Cap01 eine Verbesserung der Schaumeigenschaften in dem polyetherfreien Tensidesystem bewirkt.

Zusammenfassend kann festgestellt werden, dass Cap01 sowohl auf die Schaumqualität als auch auf das Hautgefühl während und nach der Anwendung einen deutlich positiven Einfluss in klassischen polyetherhaltigen und polyetherfreien Tensidformulierungen hat.

### Beispiel 3: Austestung der solubilisierenden Eigenschaften (in nicht erfindungsgemäßen Formulierungen)

Die solubilisierenden Eigenschaften von Cap01 wurden getestet, indem das wasserunlösliches Öl Isopropylmyristat (Evonik Goldschmidt GmbH, TEGOSOFT^{®} M) in einer Tensidlösung, bestehend aus 40 Gew.-% Natriumlaurylethersulfat (Cognis, Texapon^{®} NSO, 28 %-ig), 10 Gew.-% Cocamidopropylbetain (Evonik Goldschmidt GmbH, TEGO^{®} Betain F 50, 38%-ig) und 0,5 Gew.-% Solubilisator-Zusatz (siehe Beispiele 3a3, 3a4, 3a6 und 3a7) klar gelöst wurde. Zum Vergleich wurde das Öl in der reinen Tensidlösung (siehe Beispiele 3a1 und 3a2 Tabelle 3-1) ohne Solubilisator-Zusatz gelöst.
Als Marktstandard wurde PEG-7 Glyceryl Cocoate (Evonik Goldschmidt GmbH, TEGOSOFT^{®} GC) (siehe Beispiele 3a6, 3a7 und 3a8 Tabelle 3-1) eingesetzt.

Tabelle 3-1 gibt die Menge Isopropylmyristat (Evonik Goldschmidt GmbH, TEGOSOFT^{®} M) an, die im jeweiligen System noch klar gelöst werden konnte. Oberhalb dieser Menge kommt es zu Trübungen.
Cap01 hat eine deutliche solubilisierende Wirkung, die den Marktstandard PEG-7 Glyceryl Cocoate (Evonik Goldschmidt GmbH, TEGOSOFT^{®} GC) übertrifft.

**Tab. 3-1: Formulierungen und Ergebnisse - Solubilisierungsversuche**

| | [Gew.-%] | | | | | | | |
|---|---|---|---|---|---|---|---|---|
| **Beispiel** | **3a1*** | **3a2*** | **3a3** | **3a4** | **3a5** | **3a6*** | **3a7*** | **3a8*** |
| Texapon^{®} NSO (Cognis, INCI: Sodium Laureth Sulfate, 28%-ig) | 40 | 40 | 40 | 40 | 40 | 40 | 40 | 40 |
| TEGO^{®} Betain F 50 (Evonik Goldschmidt GmbH, INCI: Cocamidopropyl Betaine, 38%-ig) | 10 | 10 | 10 | 10 | 10 | 10 | 10 | 10 |
| TEGOSOFT^{®} M (Evonik Goldschmidt GmbH, INCI: Isopropyl Myristate) | 0,5 | 0, 6 | 1,0 | 1,5 | 1, 6 | 1,0 | 1,1 | 1,2 |
| Cap01 | | | 0,5 | 0,5 | 0,5 | | | |
| TEGOSOFT^{®} GC (Evonik Goldschmidt GmbH, INCI: PEG-7 Glyceryl Cocoate) | | | | | | 0,5 | 0,5 | 0,5 |
| Wasser | ad 100,0 | | | | | | | |
| Aussehen | klar | trüb | klar | klar | trüb | klar | klar | trüb |

| | | | | | | | | |
|---|---|---|---|---|---|---|---|---|
| * Vergleichsbeispiel | | | | | | | | |

Zusammenfassend kann gesagt werden, dass die angegebenen Beispiele die verdickende, die pflegende, schaumfördernde und die solubilisierende Wirkung von Cap01 eindeutig nachweisen, wobei die Wirksamkeit der Vergleichssubstanzen (Marktstandards) teilweise deutlich übertroffen wird.

### Beispiel 4: Weitere Formulierungsbeispiele:

Diese Beispiele zeigen exemplarische Vertreter einer Vielzahl erfindungsgemäßer Formulierungen.
Falls die Herstellung der Formulierung zuvor die getrennte Zubereitung bzw. Mischung von Formulierungsbestandteilen erfordert, wird dieses als mehrphasige Zubereitung bezeichnet.
Falls eine zweiphasige Herstellung erforderlich ist, werden die beiden Phasen mit A und B in den angegebenen Tabellen gekennzeichnet.
Bei dreiphasigen Prozessen werden die drei Phasen mit A, B und C benannt.

**Formulierungsbeispiel 1) Shampoo, PEG- & Sulfat frei**

| | |
|---|---|
| REWOTERIC^{®} AMC, Evonik Goldschmidt GmbH, 32%-ig, (INCI: Sodium Cocoamphoacetate) | 15,00% |
| REWOPOL^{®} SB F 12 P, Evonik Goldschmidt GmbH, 96%-ig, (INCI: Disodium Lauryl Sulfosuccinate) | 3,80% |
| Cap01 | 0,50% |
| Perfume | 0,30% |
| Water | 66,10% |
| TEGO^{®} Betain F 50, Evonik Goldschmidt GmbH, 38%-ig, (INCI: Cocamidopropyl Betaine) | 13,00% |
| ANTIL^{®} HS 60, Evonik Goldschmidt GmbH, (INCI: Cocamidopropyl Betaine; Glyceryl Laurate) | 1,00% |
| Citric Acid, 30-ig% | q.s. |
| Preservative | 0,30% |

**Formulierungsbeispiel 2) Mild Hair & Body Wash**

| | |
|---|---|
| Plantacare^{®} 1200 UP, Cognis, 50%-ig, (INCI: Lauryl Glucoside) | 11,40% |
| Plantacare^{®} 818 UP, Cognis, 51%-ig, (INCI: Coco Glucoside) | 5,60% |
| Water | 63,00% |
| Cap01 | 0,50% |
| TEGOSOFT^{®} LSE 65 K SOFT, Evonik Goldschmidt GmbH, (INCI: Sucrose Cocoate) | 1,50% |
| TEGO^{®} Betain F 50, Evonik Goldschmidt GmbH, 38%-ig, (INCI: Cocamidopropyl Betaine) | 18,00% |
| Perfume,preservative | q.s. |
| Citric Acid, 30% | q.s. |

**Formulierungsbeispiel 3) (nicht erfindungsgemäß) Moisturizing Body Wash**

| | | |
|---|---|---|
| A | TEXAPON^{®} NSO, Cognis, 28%-ig, (INCI: Sodium Laureth Sulfate) | 30,00% |
| | Cap01 | 0,70% |
| | Perfume | 0,30% |
| B | Water | 55,40% |
| | TEGOCEL^{®} fluid HPM 4000, Evonik Goldschmidt GmbH, (INCI: Hydroxypropyl Methylcellulose) | 1,20% |
| | TEGO^{®} Betain C 60, Evonik Goldschmidt GmbH, 46%-ig, (INCI: Cocamidopropyl Betaine) | 8,10% |
| | TEGOSOFT^{®} APM, Evonik Goldschmidt GmbH, (INCI: PPG-3 Myristyl Ether) | 1,00% |
| | TEGO^{®} Pearl N 300, Evonik Goldschmidt GmbH, (INCI: Glycol Distearate; Laureth-4; Cocamidopropyl Betaine) | 2,00% |
| | REWODERM^{®} LI S 80, Evonik Goldschmidt GmbH, (INCI: PEG-200 Hydrogenated Glyceryl Palmate; PEG-7 Glyceryl Cocoate) | 1,00% |
| | Preservative | 0, 60% |
| | Citric Acid, 30%-ig | q.s. |

**Formulierungsbeispiel 4) Clear Shower Gel for dry (nicht erfindungsgemäß)**

| | |
|---|---|
| Cap01 | 1,00% |
| TAGAT^{®} CH 40, Evonik Goldschmidt GmbH, (INCI: PEG-40 Hydrogenated Castor Oil) | 2,50% |
| Perfume | 0,30% |
| TEXAPON^{®} NSO, Cognis, 28%-ig, (INCI: Sodium Laureth Sulfate) | 42,90% |
| Water | 39,30% |
| TEGO^{®} Betain F 50, Evonik Goldschmidt GmbH, 38%-ig, (INCI: Cocamidopropyl Betaine) | 10,70% |
| LACTIL^{®}, Evonik Goldschmidt GmbH, (INCI: Sodium Lactate; Sodium PCA; Glycine; Fructose; Urea; Niacinamide; Inositol; Sodiumbenzoate; Lactic Acid) | 1,00% |
| ANTIL^{®} 171, Evonik Goldschmidt GmbH, (INCI: PEG-18 Glyceryl Oleate/Cocoate) | 2,00% |
| Preservative | 0,30% |

**Formulierungsbeispiel 5) Mild Facial Cleansing Foam (nicht erfindungsgemäß)**

| | | |
|---|---|---|
| A | Water | 82,20% |
| | TEGO^{®} Cosmo C 100, Evonik Goldschmidt GmbH, (INCI: Creatine) | 0, 25% |
| | TEGOCEL^{®} HPM 50, Evonik Goldschmidt GmbH, (INCI: Hydroxypropyl Methylcellulose) | 0,50% |
| B | TEGO^{®} Betain 810, Evonik Goldschmidt GmbH, 38%-ig, (INCI: Capryl/Capramidopropyl Betaine) | 6,60% |
| | REWOPOL^{®} SB CS 50 B, Evonik Goldschmidt GmbH, 40%-ig, (INCI: Disodium PEG-5 Laurylcitrate Sulfosuccinate; Sodium Laureth Sulfate) | 8,00% |
| | Cap01 | 0,70% |
| | Perfume | 0,25% |
| | LACTIL^{®}, Evonik Goldschmidt GmbH, (INCI: Sodium Lactate; Sodium PCA; Glycine; Fructose; Urea; Niacinamide; Inositol; Sodium benzoate; Lactic Acid) | 1,00% |
| | Panthenol | 0,20% |
| | Preservative | 0,30% |

**Formulierungsbeispiel 6) Clear Moisturizing Shower Gel (nicht erfindungsgemäß)**

| | |
|---|---|
| TEXAPON^{®} NSO, Cognis, 28%-ig, (INCI: Sodium Laureth Sulfate) | 37,00% |
| Cap01 | 1,00% |
| Perfume | 0,30% |
| Water | 42,00% |
| REWOTERIC^{®} AMC, Evonik Goldschmidt GmbH, 32%-ig, (INCI: Sodium Cocoamphoacetate) | 9,00% |
| TEGO^{®} Betain 810, Evonik Goldschmidt GmbH, 38%-ig, (INCI: Capryl/Capramidopropyl Betaine) | 7,60% |
| LACTIL^{®}, Evonik Goldschmidt GmbH, (INCI: Sodium Lactate; Sodium PCA; Glycine; Fructose; Urea; Niacinamide; Inositol; Sodiumbenzoate; Lactic Acid) | 1,00% |
| Citric Acid, 30%-ig | 1,30% |
| REWODERM^{®} LI S 80, Evonik Goldschmidt GmbH, (INCI: PEG-200 Hydrogenated Glyceryl Palmate; PEG-7 Glyceryl Cocoate) | 0,50% |
| Preservative | 0,30% |

**Formulierungsbeispiel 7) Shampoo, PEG- & sulfate free**

| | |
|---|---|
| REWOTERIC^{®} AM C, Evonik Goldschmidt GmbH, 32%-ig, (INCI: Sodium Cocoamphoacetate) | 15,00% |
| Plantapon ACG 50, Cognis (INCI: Disodium Cocoyl Glutamate) | 3,80% |
| Cap01 | 1,00% |
| Perfume | 0,30% |
| Water | 66,30% |
| TEGO^{®} Betain F 50, Evonik Goldschmidt GmbH, 38%-ig, (INCI: Cocamidopropyl Betaine) | 10,00% |
| VARISOFT^{®} PATC, Evonik Goldschmidt GmbH, (INCI: Palmitamidopropyltrimonium Chloride) | 2,30% |
| REWOMID^{®} SPA, Evonik Goldschmidt GmbH, (INCI: Isostearamide MIPA) | 1,00% |
| Preservative | 0,30% |
| Citric Acid, 30 %-ig | q.s. |

**Formulierungsbeispiel 8) Shower Gel for sensitive skin (nicht erfindungsgemäß)**

| | |
|---|---|
| TEXAPON^{®} NSO, Cognis, 28%-ig (INCI: Sodium Laureth Sulfate) | 15,00% |
| Cap01 | 0,50% |
| Perfume | 0,30% |
| PGFAC-S, Cognis (INCI: Sodium cocoyl hydrolyzed wheat protein glutamate) | 1,50% |
| REWOPOL SB CS 50 B, Evonik Goldschmidt GmbH, 40%-ig, (INCI: Disodium PEG-5 Laurylcitrate Sulfosuccinate; Sodium Laureth Sulfate) | 7,50% |
| Water | 60,10% |
| TEGO^{®} Betain F 50, Evonik Goldschmidt GmbH, 38%-ig, (INCI: Cocamidopropyl Betaine) | 9,00% |
| TEGO^{®} Betain 810, Evonik Goldschmidt GmbH, 38%-ig, (INCI: Capryl/Capramidopropyl Betaine) | 4,00% |
| ANTIL^{®} 200, Evonik Goldschmidt GmbH, (INCI: PEG-200 Hydrogenated Glyceryl Palmate; PEG-7 Glyceryl Cocoate) | 1,80% |
| Preservative | 0,30% |

**Formulierungsbeispiel 9) Shampoo, PEG- & sulfate free**

| | | |
|---|---|---|
| A | REWOTERIC^{®} AMC, Evonik Goldschmidt GmbH, 32%-ig, (INCI: Sodium Cocoamphoacetate) | 20,00% |
| | REWOPOL^{®} SB F 12 P, Evonik Goldschmidt, 96%-ig, (INCI: Disodium Lauryl Sulfosuccinate) | 5,90% |
| | Cap01 | 0,70% |
| B | Water | 66,20% |
| | Citric Acid, 30%-ig | 3,60% |
| C | ANTIL^{®} HS 60, Evonik Goldschmidt GmbH, (INCI: Cocamidopropyl Betaine; Glyceryl Laurate) | 3,00% |
| | Preservative | 0,60% |

**Formulierungsbeispiel 10) Mild Body Wash (nicht erfindungsgemäß)**

| | | |
|---|---|---|
| A | TEXAPON^{®} NSO Cognis 28%-ig, (INCI: Sodium Laureth Sulfate | 30,00% |
| | Cap01 | 0,50% |
| | ABIL^{®} B 8832, Evonik Goldschmidt GmbH, (INCI: Bis-PEG/PPG-20/20 Dimethicone) | 0,30% |
| | Perfume | 0,30% |
| B | Water | 53,00% |
| | TEGOCEL^{®} fluid HPM 4000, Evonik Goldschmidt GmbH, (INCI: Hydroxypropyl Methylcellulose) | 1,20% |
| | Citric Acid Monohydrate | 0,50% |
| | REWOTERIC^{V} AM C, Evonik Goldschmidt GmbH, 32%-ig, (INCI: Sodium Cocoamphoacetate) | 10,00% |
| | TEGO^{®} Pearl N 300, Evonik Goldschmidt GmbH, (INCI: Glycol Distearate; Laureth-4; Cocamidopropyl Betaine) | 2,00% |
| | REWODERM^{®} LI S 80, Evonik Goldschmidt GmbH, (INCI: PEG-200 Hydrogenated Glyceryl Palmate; PEG-7 Glyceryl Cocoate) | 1,60% |
| | Preservative | 0,60% |
| | Citric Acid, 30%-ig | q.s. |

**Formulierungsbeispiel 11) Sprayable Hairmilk, PEG-free**

| | | |
|---|---|---|
| A | Water | 95,30% |
| | Lactic Acid, 80%-ig | 0,40 |
| B | TEGO^{®} AMID S 18, Evonik Goldschmidt GmbH, (INCI: Stearamidopropyl Dimethylamine) | 1,20% |
| | TEGIN^{®} G 1100 Pellets, Evonik Goldschmidt GmbH, (INCI: Glycol Distearate) | 0,60% |
| | TEGO^{®} Care PS, Evonik Goldschmidt GmbH, (INCI: Methyl Glucose Sesquistearate) | 1,20% |
| | TEGOSOFT^{®} DEC, Evonik Goldschmidt GmbH, (INCI: Diethylhexyl Carbonate) | 0,30% |
| | Cap01 | 1,00% |
| | Perfume, preservative | q.s. |

**Formulierungsbeispiel 12) Body cleansing Foam (nicht erfindungsgemäß)**

| | |
|---|---|
| TEXAPON^{®} NSO, Cognis, 28%-ig (INCI: Sodium Laureth Sulfate) | 14% |
| Perfume | 0.3% |
| Cap01 | 0.5% |
| REWOTERIC^{®} AM C, Evonik Goldschmidt GmbH, 32%-ig (INCI: Sodium Cocoamphoacetate) | 8% |
| Water | 75.2% |
| TEGOCEL^{®} HPM 50, Evonik Goldschmidt GmbH (INCI: Hydroxypropyl Methylcellulose) | 0.5% |
| LACTIL^{®}, Evonik Goldschmidt GmbH (INCI: Sodium Lactate; Sodium PCA; Glycine; Fructose; Urea; Niacinamide; Inositol; Sodium benzoate; Lactic Acid) | 1% |
| Citric Acid Monohydrate | 0.5% |

**Formulierungsbeispiel 13) Formulierungsbeispiel Clear Conditioning Shampoo (nicht erfindungsgemäß)**

| | |
|---|---|
| TEXAPON^{®} NSO, Cognis, 28%-ig (INCI: Sodium Laureth Sulfate) | 32,00% |
| VARISOFT^{®} PATC, Evonik Goldschmidt GmbH(INCI: Palmitamidopropyltrimonium Chloride) | 1,50% |
| REWODERM^{®} LI S 80, Evonik Goldschmidt GmbH (INCI: PEG-200 Hydrogenated Glyceryl Palmate; PEG-7 Glyceryl Cocoate) | 2,00% |
| Cap01 | 0,50% |
| Perfume | 0,25% |
| Water | 54,05 |
| TEGO^{®} Cosmo C 100, Evonik Goldschmidt GmbH, (INCI: Creatine) | 1**,**00% |
| Jaguar C-162, Rhodia (INCI: Hydroxypropyl Guar Hydroxypropyltrimonium Chloride) | 0,20 |
| TEGO^{®} Betain F 50, Evonik Goldschmidt GmbH, 38%-ig (INCI: Cocamidopropyl Betaine) | 8,00% |
| NaCl | 0,50% |
| Preservative | q.s. |

**Formulierungsbeispiel 14) Formulierungsbeispiel Pearlized Shampoo (nicht erfindungsgemäß)**

| | |
|---|---|
| TEXAPON^{®} NSO, Cognis, 28%-ig (INCI: Sodium Laureth Sulfate) | 32,00% |
| Cap01 | 0,50% |
| Perfume | 0,25% |
| Water | 55,25 |
| TEGO^{®} Betain F 50, Evonik Goldschmidt GmbH, 38%-ig (INCI: Cocamidopropyl Betaine) | 8,00% |
| TEGO^{®} Pearl N 300 Evonik Goldschmidt GmbH (INCI: Glycol Distearate; Laureth-4; Cocamidopropyl Betaine) | 2,00% |
| ANTIL^{®} 171 Evonik Goldschmidt GmbH (INCI: PEG-18 Glyceryl Oleate/Cocoate) | 1,50% |
| NaCl | 0,50% |
| Preservative | q.s. |

**Formulierungsbeispiel 15) Formulierungsbeispiel Rinse-Off Conditioner**

| | |
|---|---|
| Water | 90,20% |
| VARISOFT^{®} EQ 65, Evonik Goldschmidt GmbH (INCI: Distearyl Dimonium Chloride, Cetearyl Alcohol) | 2,00% |
| VARISOFT^{®} BT 85, Evonik Goldschmidt GmbH (INCI: Behentrimonium Chloride) | 2,00% |
| Cap01 | 0,80% |
| TEGO^{®} Alkanol 1618, Evonik Goldschmidt GmbH (INCI: Cetearyl Alcohol) | 5,00 |
| Preservative, Perfume | q.s. |

**Formulierungsbeispiel 16) Formulierungsbeispiel Clear Conditioning Shampoo (nicht erfindungsgemäß)**

| | |
|---|---|
| TEXAPON^{®} NSO, Cognis, 28%-ig (INCI: Sodium Laureth Sulfate) | 32,00% |
| ANTIL^{®} 200, Evonik Goldschmidt GmbH (INCI: PEG-200 Hydrogenated Glyceryl Palmate; PEG-7 Glyceryl Cocoate) | 2,00% |
| Ca01 | 1,00% |
| Perfume | 0,25% |
| Water | 56,25 |
| Polymer JR 400, Amerchol (INCI: Polyquaternium-10) | 0,20 |
| TEGO^{®} Betain F 50, Evonik Goldschmidt GmbH, 38%-ig (INCI: Cocamidopropyl Betaine) | 8,00% |
| NaCl | 0,30% |
| Preservative | q.s. |

## Patentansprüche

1. Formulierungen zur Reinigung und Pflege von menschlichen oder tierischen Körperteilen enthaltend Sorbitancarbonsäureester und ein weiteres Tensid,
**dadurch gekennzeichnet, dass**
der Carbonsäureanteil des Sorbitancarbonsäureesters sich ableitet von einer Carbonsäure enthaltend 6 bis 10 Kohlestoff-Atome und
die Sorbitancarbonsäureester eine Hydroxylzahl (OH-Zahl) von größer 350 aufweisen, **dadurch gekennzeichnet, dass** die Formulierung im Wesentlichen frei von Polyethern und Polyether enthaltenden Verbindungen ist und dass 1,2 bis 2 Mol Carbonsäure pro Mol Sorbitol,
von dem sich der Sorbitancarbonsäureester ableitet, eingeestert sind.

2. Formulierung gemäß Anspruch 1, **dadurch gekennzeichnet, dass** die Formulierung von 0,01 Gew.-% bis 10 Gew.-% Sorbitancarbonsäureester bezogen auf die Gesamtformulierung enthält.

3. Formulierung gemäß mindestens einem der Ansprüche 1 oder 2, **dadurch gekennzeichnet, dass** die Formulierung eine wässrige tensidische Formulierung ist.

4. Formulierung gemäß mindestens einem der Ansprüche 1 bis 3, **dadurch gekennzeichnet, dass** die enthaltenen Sorbitancarbonsäureester eine Säurezahl von kleiner 20 aufweisen.

5. Formulierung gemäß mindestens einem der Ansprüche 1 bis 4, **dadurch gekennzeichnet, dass** die enthaltenen Sorbitancarbonsäureester eine Iodzahl von kleiner 30 aufweisen.

6. Formulierung gemäß mindestens einem der Ansprüche 1 bis 5, **dadurch gekennzeichnet, dass** die enthaltenen Sorbitancarbonsäureester eine Viskosität von 100 bis 20000 mPa·s aufweisen.

7. Formulierung gemäß mindestens einem der Ansprüche 1 bis 6, **dadurch gekennzeichnet, dass** die enthaltenen Sorbitancarbonsäureester erhältlich sind durch ein Verfahren umfassend die Verfahrensschritte
A) Dehydratisierung von Sorbitol,
B) Umsetzung des dehydratisierten Sorbitols mit mindestens einer Verbindung ausgewählt aus der Gruppe umfassend Carbonsäuren enthaltend 6 bis 10 Kohlenstoffatome, Carbonsäureester oder Carbonsäureestermischungen, bei denen die Carbonsäurekomponente 6 bis 10 Kohlenstoffatome enthält, und gegebenenfalls
C) Isolierung von gebildetem Sorbitanester aus Verfahrensschritt B).

8. Verwendung von Sorbitancarbonsäureestern,
bei denen der Carbonsäureanteil sich ableitet von einer Carbonsäure enthaltend 6 bis 10 Kohlestoff-Atome
und
die eine Hydroxylzahl (OH-Zahl) von größer 350 aufweisen,
als Viskositätsregler, Pflegewirkstoff, Schaum-Booster oder Solubilisator in polyetherfreien tensidischen Formulierungen, **dadurch gekennzeichnet, dass** 1,2 bis 2 Mol Carbonsäure pro Mol Sorbitol, von dem sich der Sorbitancarbonsäureester ableitet, eingeestert sind.

## Claims

1. Formulations for the cleansing and care of human or animal body parts containing sorbitan carboxylic acid esters and a further surfactant,
**characterized in that**
the carboxylic acid component of the sorbitan carboxylic acid ester is derived from a carboxylic acid containing 6 to 10 carbon atoms and
the sorbitan carboxylic acid esters have a hydroxyl number (OH number) of greater than 350,
**characterized in that** the formulation is essentially free of polyethers and polyether-containing compounds and **in that** 1.2 to 2 mol of carboxylic acid per mole of sorbitol from which the sorbitan carboxylic acid ester is derived are esterified.

2. Formulation according to Claim 1, **characterized in that** the formulation contains from 0.01% by weight to 10% by weight of sorbitan carboxylic acid ester based on the total formulation.

3. Formulation according to at least one of Claims 1 or 2, **characterized in that** the formulation is an aqueous surfactant formulation.

4. Formulation according to at least one of Claims 1 to 3, **characterized in that** the sorbitan carboxylic acid esters contained have an acid number of less than 20.

5. Formulation according to at least one of Claims 1 to 4, **characterized in that** the sorbitan carboxylic acid esters contained have an iodine number of less than 30.

6. Formulation according to at least one of Claims 1 to 5, **characterized in that** the sorbitan carboxylic acid esters contained have a viscosity of 100 to 20 000 mPa·s.

7. Formulation according to at least one of Claims 1 to 6, **characterized in that** the sorbitan carboxylic acid esters contained are obtainable by a process comprising the process steps
A) dehydration of sorbitol,
B) reaction of the dehydrated sorbitol with at least one compound selected from the group comprising carboxylic acids containing 6 to 10 carbon atoms, carboxylic acid esters or carboxylic acid ester mixtures, in which the carboxylic acid component contains 6 to 10 carbon atoms, and optionally
C) isolation of formed sorbitan esters from process step B).

8. Use of sorbitan carboxylic acid esters,
in which the carboxylic acid content is derived from a carboxylic acid containing 6 to 10 carbon atoms
and
which have a hydroxyl number (OH number) of greater than 350,
as a viscosity regulator, care active ingredient, foam booster or solubilizer in polyether-free surfactant formulations, **characterized in that** 1.2 to 2 mol of carboxylic acid per mole of sorbitol from which the sorbitan carboxylic acid ester is derived are esterified.

## Revendications

1. Compositions destinées au nettoyage et au soin de parties de corps humaines ou animales, contenant un ester de sorbitane avec un acide carboxylique et un autre tensioactif,
**caractérisées en ce que**
le fragment d'acide carboxylique de l'ester de sorbitane avec un acide carboxylique dérive d'un acide carboxylique contenant de 6 à 10 atomes de carbone et les esters de sorbitane avec un acide carboxylique présentent un indice d'hydroxyle (indice d'OH) supérieur à 350, **caractérisées en ce que** la composition est essentiellement exempte de polyéthers et de composés contenant des polyéthers et **en ce que** 1,2 à 2 moles d'acide carboxylique par mole de sorbitol, dont dérive l'ester de sorbitane avec un acide carboxylique, sont incorporées par estérification.

2. Composition selon la revendication 1, **caractérisée en ce que** la composition contient de 0,01 % en poids à 10 % en poids d'ester de sorbitane avec un acide carboxylique, par rapport à la composition totale.

3. Composition selon au moins l'une des revendications 1 et 2, **caractérisée en ce que** la composition est une composition aqueuse tensioactive.

4. Composition selon au moins l'une des revendications 1 à 3, **caractérisée en ce que** les esters de sorbitane avec un acide carboxylique contenus présentent un indice d'acide inférieur à 20.

5. Composition selon au moins l'une des revendications 1 à 4, **caractérisée en ce que** les esters de sorbitane avec un acide carboxylique contenus présentent un indice d'iode inférieur à 30.

6. Composition selon au moins l'une des revendications 1 à 5, **caractérisée en ce que** les esters de sorbitane avec un acide carboxylique contenus présentent une viscosité de 100 à 20 000 mPa.s.

7. Composition selon au moins l'une des revendications 1 à 6, **caractérisée en ce que** les esters de sorbitane avec un acide carboxylique contenus peuvent être obtenus par un procédé comprenant les étapes de processus
A) déshydratation de sorbitol,
B) mise en réaction du sorbitol déshydraté avec au moins un composé choisi dans le groupe comprenant des acides carboxyliques contenant de 6 à 10 atomes de carbone, des esters d'acides carboxyliques ou des mélanges d'esters d'acides carboxyliques, dans lesquels le composant d'acide carboxylique contient de 6 à 10 atomes de carbone, et éventuellement
C) isolement de l'ester de sorbitane formé, provenant de l'étape B) du procédé.

8. Utilisation d'esters de sorbitane avec un acide carboxylique,
dans lesquels le fragment d'acide carboxylique dérive d'un acide carboxylique contenant de 6 à 10 atomes de carbone
et
qui présentent un indice d'hydroxyle (indice d'OH) supérieur à 350,
en tant que régulateurs de viscosité, substance active de soin, renforçateur de mousse ou solubilisant dans des compositions tensioactives sans polyéthers, **caractérisée en ce que** 1,2 à 2 moles d'acide carboxylique par mole de sorbitol, dont dérive l'ester de sorbitane avec un acide carboxylique, sont incorporées par estérification.
